**Europäisches Patentamt**

**European Patent Office**

**Office européen des brevets**

(19)

(11) Veröffentlichungsnummer: **0 163 814**
**B1**

(12) # EUROPÄISCHE PATENTSCHRIFT

(45) Veröffentlichungstag der Patentschrift:
**07.01.88**

(51) Int. Cl.⁴: **A 61 C 19/06**, A 61 N 1/42

(21) Anmeldenummer: **85102257.4**

(22) Anmeldetag: **28.02.85**

(54) Zahnpflegegerät.

(30) Priorität: **28.04.84 DE 8413117 U**
**06.11.84 DE 8432394 U**
**30.10.84 DE 8431762 U**

(43) Veröffentlichungstag der Anmeldung:
**11.12.85 Patentblatt 85/50**

(45) Bekanntmachung des Hinweises auf die Patenterteilung:
**07.01.88 Patentblatt 88/1**

(84) Benannte Vertragsstaaten:
**AT BE CH DE FR GB IT LI LU NL SE**

(56) Entgegenhaltungen:
**EP - A - 0 052 064**
**DE - A - 2 022 360**
**DE - A - 2 827 990**
**DE - A - 3 221 544**
**DE - A - 3 231 837**
**GB - A - 2 117 230**

(73) Patentinhaber: **Pose, Wolfgang, Dr., Eppendorfer
Landstrasse 44, D-2000 Hamburg 20 (DE)**

(72) Erfinder: **Pose, Wolfgang, Dr., Eppendorfer
Landstrasse 44, D-2000 Hamburg 20 (DE)**

(74) Vertreter: **Patentanwälte Dipl.-Ing. J. Richter Dipl.-Ing. F.
Werdermann, Neuer Wall 10, D-2000 Hamburg 36 (DE)**

**Beschreibung**

Die Erfindung betrifft ein Zahnpflegegerät, bestehend aus einer stabartigen Handhabe mit einem einendseitig vorgesehenen Gehäuse mit einer in diesem angeordneten Magnetfeld-Erzeugungseinrichtung.

Alle Elemente, Substanzen, chemische Verbindungen, Medikamente und auch lebende Organismen haben ihre eigenspezifische energetisch-informative Abstrahlung, die sich entweder günstig oder belastend auf biologische Systeme, wie Mensch – Tier – Pflanze auswirkt. Hierbei kommt es auf die Wellenlängen, auf die Polarisationsrichtung, d.h. links- oder rechtspolarisiert und auf die Intensität an. So haben alle Schadstoffe, und dazu müssen auch die meisten zahnärztlichen Werkstoffe, wie z.B. Amalgam, gerechnet werden, spezifische energetische Abstrahlungen, die als Schadstoff-Information auf die körpereigenen Regelmechanismen biologischer Systeme einwirken und vom Organismus gespeichert werden. Es kann daher infolge Langzeiteinwirkung zu Summations- und Kumulationseffekten kommen, die die Ursache für chronisch-schleichende Erkrankungen mit einer sehr vielgestalteten Symptomatik sein können.

Es ist bekannt, dass von den verschiedensten Substanzen und Elementen von magnetischen Zustandsänderungen der Materie herrührende Temperaturänderungen auftreten, die auf einer mit der Magnetisierungsänderung verbundenen Veränderung des Ordnungszustandes beruhen, die zu einer Änderung der Entropie des magnetischen Systems führt. So ist es auch bekannt, dass jedes Element und auch jede Substanz eine ganz spezifische Strahlung aussendet, die unter die physikalischen Eigenschaften eines Elementes fällt, während daneben auch noch chemische Eigenschaften eines jeden Elementes bekannt sind. Jedes Element hat hiernach eine energetische Abstrahlung bestimmter Wellenlänge, Polarität und Intensität. Besonders ausgeprägt ist die spezifische Strahlung bei radioaktiven Elementen, wobei in besonderen Fällen die chemische Eigenart eines Elementes als beständige Einheit hinter der physikalischen Veränderlichkeit gänzlich zurücktritt, so dass davon ausgegangen werden muss, dass die Strahlung bei derartigen Stoffen etwas Selbständiges, von den chemischen Eigenschaften Losgelöstes ist. Diese Umstände berücksichtigend hat sich die Strahlentherapie entwickelt, und zwar basierend auf den magnetischen Feldlinien und deren Anwendungsgebiet die Magnetfeld-Therapie.

Nach der EP-0052064 ist es bekannt, in dem Borsten tragenden Körper von Zahnbürsten Permanentmagnete als Magnetfeld-Erzeugungseinrichtung anzuordnen. Des weiteren ist nach der GB-A-2117230 eine Zahnbürste bekannt, bei der in dem die Borsten tragendenden Borstenkörper sowohl ein einziger als auch eine grössere Anzahl von Permanentmagneten angeordnet sein kann.

Mit ein Magnetfeld erzeugenden Zahnbürsten wird den Benutzern derartiger Zahnbürsten ein Zahnpflegegerät zur Verfügung gestellt, mit dem bei der Reinigung der Zähne und bei der Massage des Zahnfleisches gleichzeitig eine Magnetfeld-Therapie durchführbar iat, die auf der Erkenntnis beruht, dass, wenn der menschliche Körper oder einzelne Körperteile in den Wirkungsbereich von Magnetfeldern gebracht werden, dann die magnetischen Feldlinien den Körper oder die Körperteile vollständig mit absoluter Tiefenwirkung durchdringen, wobei die magnetischen Feldlinien im Frequenztakt auf die für die Zellfunktion wichtigen Ionen auftreffen, so dass durch eine Behandlung mit einem Magnetfeld angegriffenes Zahnfleisch gekräftigt und regeneriert werden kann. Hinzu kommt, dass durch das beim Reinigen der Zähne gleichzeitig vom Permanentmagneten der Zahnbürste erzeugte Magnetfeld ein Wachstum von Bakterien gehemmt wird. Derartige, mit einem Permanentmagneten versehene Zahnbürsten sind jedoch nicht als zahnärztliches Gerät verwendbar und durch die Verwendung eines feststehend angeordneten Permanentmagneten in dem die Borsten tragenden Bürstenkörper der Zahnbürste ist es nicht möglich, ein ständig wechselndes Magnetfeld auszubilden, um eine Intensivierung und Ausstrahlung des Magnetfeldes in einem grossen Bereich zu erreichen.

Die Erfindung löst die Aufgabe, ein Zahnpflegegerät zu schaffen, mit dem unter Anwendung der intraoralen Magnetfeld-Therapie auch eine Inaktivierung bzw. Neutralisierung der energetischen Schadstoff-Abstrahlungen aller im Mund befindlichen zahnärztlichen Werkstoffe, wie z.B. Silber- oder Kupfer-Amalgam, Silikatzemente, Zemente für Unterfüllungen, Composites, Zahnfüllungen aus selbsthärtenden Kunststoffen, Wurzelfüllmaterialien, Metallegierungen aller Art für Zahnersatzarbeiten u.dgl. in einfachster Weise möglich ist und das darüber hinaus auch als zahnärztliches Gerät einsetzbar ist.

Diese Aufgabe wird bei einem Zahnpflegegerät gemäss der eingangs beschriebenen Art durch die im Patentanspruch 1 gekennzeichneten Merkmale gelöst.

Mit einem derart ausgebildeten Zahnpflegegerät ist es möglich, durch intraorale Magnetfeld-Therapie energetische Schadstoff-Abstrahlungen aller im Mund befindlicher zahnärztlicher Werkstoffe zu inaktivieren bzw. zu neutralisieren. Überraschend hat es sich gezeigt, dass durch umfangreiche Testversuche mit Hilfe der Elektroakupunktur an vielen Testpersonen festgestellt worden ist, dass nach Einwirken eines Magnetfeldes oder von Magnetfeldern die energetischen Schadstoff-Abstrahlungen inaktiviert und damit unwirksam gemacht werden konnten. Es hat sich ferner gezeigt, dass beim Herausbohren von Amalgam-Füllungen nach Durchführung der intraoralen Magnetfeld-Therapie keine Schadwirkung durch den Bohrstaub mehr auf Behandler und Patienten testbar war. Mögliche Unverträglichkeitsreaktionen, die durch zahnärztliche Werkstoffe bedingt sind, werden durch die intraorale Magnetfeld-Therapie mit dem Zahnpflegegerät ausgeschaltet und verhindert.

Weitere vorteilhafte Ausgestaltungen der Erfindung gehen aus den Unteransprüchen hervor.

Im folgenden wird der Gegenstand der Erfindung in den Zeichnungen erläutert. Es zeigen:

Fig. 1 in einer Ansicht von oben ein stabförmiges Zahnpflegegerät mit einem rotierenden Magneten als Magnetfeld-Erzeugungseinrichtung,

Fig. 2 einen senkrechten Schnitt gemäss Linie II-II in Fig. 1 und

Fig. 3 teils in Ansicht, teils in einem senkrechten Längsschnitt eine weitere Ausführungsform eines Zahnpflegegerätes mit einer Magnetfeld-Erzeugungseinrichtung und einem im vorderen Gerätebereich angeordneten, auswechselbaren Mundschutz.

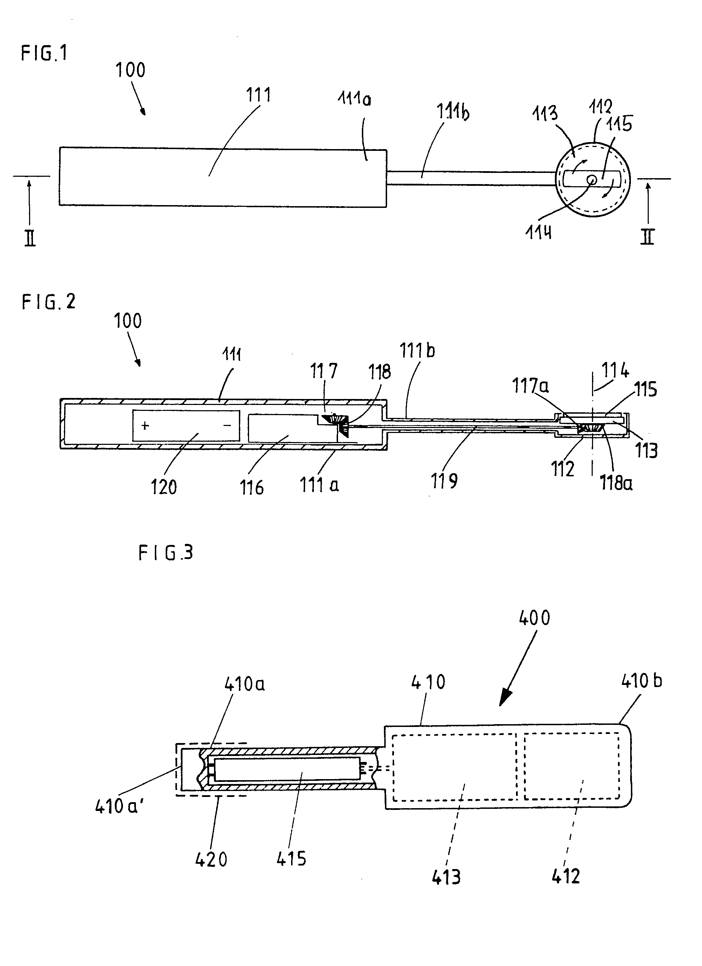

Das in Fig. 1 mit 100 bezeichnete Zahnpflegegerät, welches auch als zahnärztliches Gerät Verwendung finden kann, besteht aus einer stabartigen Handhabe 111, die gehäuseartig ausgebildet und mit einer verschliessbaren Öffnung versehen ist, um in den Innenraum des Gehäuses zu gelangen. An dem Ende 111a dieser Handhabe 111 ist ein verjüngter, stabartiger Gehäuseabschnitt 111b vorgesehen, der ein scheibenförmiges Gehäuse 112 trägt, in dem eine Scheibe 113 drehbar gelagert ist. Diese Scheibe 113 ist um die Achse 114 mittels einer im Innenraum der Handhabe 111 untergebrachten Antriebseinrichtung 116 antreibbar. Die rotierende Scheibe 113 trägt mindestens einen Stabmagneten 115. Auf der Scheibe 113 können jedoch auch mehrere radial angeordnete Stabmagnete vorgesehen sein. Das Gehäuse besteht aus solchen Werkstoffen, dass keine Beeinträchtigung der von dem Magneten abgegebenen magnetischen Feldlinien erfolgt.

Der Antrieb der Scheibe 113 mit dem Stabmagneten 115 erfolgt von der Antriebseinrichtung 116 aus über eine Antriebswelle 119 unter Zwischenschaltung von Kegelschraubradpaaren 117, 118 und 117a, 118a. Die Antriebseinrichtung 116 ist als Elektromotor ausgebildet. Die Stromversorgung der Antriebseinrichtung 116 erfolgt über eine im Innenraum der Handhabe 111 angeordnete Batterie 120, jedoch ist es auch möglich, andere Stromquellen für den Antrieb des Elektromotors der Antriebseinrichtung 116 heranzuziehen. Auch andere, in an sich bekannter Weise ausgebildete Antriebseinrichtungen können Verwendung finden. So besteht beispielsweise die Möglichkeit, vorhandene Druckluft für den Antrieb einer entsprechend ausgebildeten Antriebseinrichtung zu verwenden.

Der Dauermagnet kann als Stab-, Ring- oder Scheibenmagnet ausgebildet sein.

Bei dem in Fig. 1 und 2 dargestellten zahnärztlichen Gerät 100 ist mindestens ein Stabmagnet 115 vorgesehen, der in rotierende Bewegung versetzt wird.

Auch das in Fig. 3 dargestellte Zahnpflegegerät findet als zahnärztliches Gerät Verwendung und ist mit 400 bezeichnet; jedoch auch eine Anwendung dieses Gerätes nur zur Pflege der Zähne ist möglich.

Dieses in Fig. 3 dargestellte Gerät 400 besteht aus einem Gehäuse 410 mit einer in dessen Innenraum angeordneten Magnetfeld-Erzeugungseinrichtung 415, die als rotierender Dauermagnet oder als Elektromagnet ausgebildet ist. Vorzugsweise ist bei dieser Ausführungsform die Magnetfeld-Erzeugungseinrichtung 415 in einem fühlerartig ausgebildeten Gehäuseabschnitt 410a angeordnet, wobei im Innenraum des verbleibenden Gehäuseabschnittes 410b der Antriebsmotor 413 und eine Stromquelle 412 angeordnet sind, wobei es sich bei letzterer um eine Batterie od.dgl. handelt.

An dem vorderen freien Ende 410a' des Gehäuseabschnittes 410a ist ein vorzugsweise waschbarer und/oder sterilisierbarer Mundschutz 420 vorgesehen, der an dem Gerätegehäuse auswechselbar mittels eines Sprengringes oder anderer geeigneter Befestigungseinrichtungen gehalten ist. Dieser Mundschutz 420 besteht aus einem auf das freie Ende 410a' des Gehäuseabschnittes 410a aufziehbaren, z.B. kappenförmigen, Zuschnitt, aus geeigneten Geweben oder aus natürlichen oder Kunststoff-Fasern.

Bei allen Ausführungsformen des auch als zahnärztliches Gerät einsetzbaren Zahnpflegegerätes können die verwendeten Magnetfeld-Erzeugungseinrichtungen entweder als rotierende Dauermagneten oder als Elektromagneten ausgebildet sein. Auch andersartig ausgebildete Einrichtungen zur Erzeugung eines magnetischen Wechselfeldes können zur Anwendung gelangen.

## Patentansprüche

1. Zahnpflegegerät, bestehend aus einer stabartigen Handhabe (111; 410) mit einem einendseitig vorgesehenen Gerätegehäuse (112; 410a) mit einer in diesem angeordneten Magnetfeld-Erzeugungseinrichtung (115; 415), dadurch gekennzeichnet, dass die Magnetfeld-Erzeugungseinrichtung (115; 415) in dem Gerätegehäuse (112; 410a) um eine Achse (114) rotierbar angeordnet ist und dass die Magnetfeld-Erzeugungseinrichtung mittels einer in dem Handhabengehäuse (111; 410) angeordneten, batterieangetriebenen Antriebseinrichtung (116; 413) umlaufend antreibbar ist.

2. Zahnpflegegerät nach Anspruch 1, dadurch gekennzeichnet, dass in dem Gerätegehäuse (112) eine um eine quer zur Handhabenlängsachse verlaufende Achse (114) rotierbare Scheibe (113) mit mindestens einem auf dieser angeordneten Stabmagneten (115) angeordnet ist.

3. Zahnpflegegerät nach Anspruch 1 und 2, dadurch gekennzeichnet, dass das Gerätegehäuse (112) scheibenartig ausgebildet ist.

4. Zahnpflegegerät nach Anspruch 1 bis 3, dadurch gekennzeichnet, dass die Magnetfeld-Erzeugungseinrichtung als Dauermagnet (115; 415) oder als Elektromagnet ausgebildet ist.

5. Zahnpflegegerät nach Anspruch 1, dadurch gekennzeichnet, dass das Gerät (400) aus einem stabartigen Gerätegehäuse (410) mit einer Magnetfeld-Erzeugungseinrichtung (415) besteht, die als rotierender Dauermagnet oder Elektromagnet ausgebildet ist, wobei die Magnetfeld-Erzeugungseinrichtung (415) in einem fühlerartig ausgebildeten Abschnitt (410a) des Gerätegehäuses (410) angeordnet ist und wobei im Innenraum des verbleibenden Gehäuseabschnittes (410b) der Antriebsmotor (413) und eine Stromquelle, wie Batterie od.dgl., (412) angeordnet sind.

6. Zahnpflegegerät nach Anspruch 5, dadurch gekennzeichnet, dass das vordere freie Ende (410a') des Gehäuseabschnittes (410a) einen vorzugsweise

waschbaren und/oder sterilisierbaren Mundschutz (420) trägt, der an dem Gerätegehäuse (410a) auswechselbar angeordnet ist.

## Claims

1. Dental care device comprising one rod-shaped handle (111; 410), having on the one end a device casing (112, 410a) in which is arranged a means (115; 415) for producing a magnetic field, characterized in that the means (115; 415) for producing the magnetic field is positioned in the casing, so as to be able to rotate around an axle (114) and that the means for producing the magnetic field can be driven rotatingly by means of a batterie controlled drive (116; 413) which is positioned in the casing (111; 410) of the handle.

2. Dental care device according to claim 1, characterized in that in the device casing (112) a rotatable disk (113) is arranged on an axle (114) which runs in transverse direction to the longitudinal axle of the handle with at least one bar magnet (115) positioned on this.

3. Dental care device according to claims 1 and 2, characterized in that the device casing (112) has the form of a disk.

4. Dental care device according to claims 1 to 3, characterized in that the means for producing a magnetic field is a permanent magnet or electric magnet.

5. Dental care device according to claim 1, characterized in that the device (400) consists of a bar-shaped device casing (410) with a means (415) for producing a magnetic field, which is a rotating permanent magnet or electric magnet, the means (415) for producing a magnetic field being located in a section (410a) shaped in the form of a sensor, of the casing (410) and whereby the inner room of the remaining section of the casing (410b) accommodates the drive motor (413) and the power source, such as a battery or similar (412).

6. Dental care device according to claim 5, characterized in that the free front end (410a') of the section of the casing (410a) has, preferably, a washable and/or sterilizable mouth mask (420), which is positioned so as to be replaceable on the device casing (410a).

## Revendications

1. Dispositif de soins dentaires, composé d'un manche (111, 410) du type tige avec un boîtier du dispositif de soins (112, 410a) prévu à l'une de ses extrémités avec un dispositif de génération de champ magnétique (115, 415) placé dans ce boîtier, caractérisé en ce que le dispositif de génération de champ magnétique (115, 415) est placé dans le boîtier du dispositif de soins (112, 410a) de manière à être rotatif autour d'un axe (114) et que la rotation du dispositif de génération de champ magnétique peut être entraînée par un dispositif d'entraînement (116, 413) à piles, placé dans le boîtier du manche (111, 410).

2. Dispositif de soins dentaires selon la revendication 1, caractérisé en ce qu'un disque rotatif (113) autour d'un axe (114) situé transversalement par rapport à l'axe longitudinal du manche qui comprend au moins un aimant droit (115) sur le disque est placé dans le boîtier du dispositif de soins (112).

3. Dispositif de soins dentaires selon les revendications 1 et 2, caractérisé en ce que le boîtier du dispositif (112) est formé à la façon d'un disque.

4. Dispositif de soins dentaires selon les revendications 1 à 3, caractérisé en ce que le dispositif de génération du champ magnétique (115, 415) est configuré comme un aimant permanent ou comme un électro-aimant.

5. Dispositif de soins dentaires selon la revendication 1, caractérisé en ce que le dispositif (400) consiste en un boîtier de dispositif du type tige (410) avec un dispositif de génération de champ magnétique (415) qui est formé comme un aimant permanent rotatif ou comme un électro-aimant, le dispositif de génération du champ magnétique (415) étant placé dans une portion (410a) du boîtier du dispositif (410) configurée à la manière d'un palpeur et le moteur d'entraînement (413) et une source de courant (412), telle qu'une pile ou équivalent, étant placés à l'intérieur de la portion de boîtier restante (410b).

6. Dispositif de soins dentaires selon la revendication 5, caractérisé en ce que l'extrémité antérieure libre (410a') de la portion du boîtier (410a) porte une protection buccale (420) qui, de préférence, peut être lavée et/ou stérilisée et qui est placée sur le boîtier du dispositif (410a) de manière à pouvoir être remplacée.

FIG.1

100

111    111a    111b    113    112    115

II    II

114

FIG.2

100

111    117    118    111b    117a    114    115

+    −

120    116    111a    119    112    113    118a

FIG.3

410a    410    400    410b

410a′

420    415    413    412